(19) Europäisches Patentamt
European Patent Office
Office européen des brevets

(11) Veröffentlichungsnummer: **0 300 155**
**A2**

(12) # EUROPÄISCHE PATENTANMELDUNG

(21) Anmeldenummer: 88107985.9

(22) Anmeldetag: 03.03.86

(51) Int. Cl.4: C07C 87/34, C07C 61/15, C07C 117/04, C07C 119/045

(30) Priorität: 16.03.85 DE 3509546

(43) Veröffentlichungstag der Anmeldung: 25.01.89 Patentblatt 89/04

(60) Veröffentlichungsnummer der früheren Anmeldung nach Art. 76 EPÜ: 0 198 192

(84) Benannte Vertragsstaaten: AT BE CH DE FR GB IT LI NL SE

(71) Anmelder: **BAYER AG**
**Konzernverwaltung RP Patentabteilung**
**D-5090 Leverkusen 1 Bayerwerk(DE)**

(72) Erfinder: **Schriewer, Michael, Dr.**
**Heymannstrasse 36**
**D-5090 Leverkusen 1(DE)**
Erfinder: **Grohe, Klaus, Dr.**
**Am Wasserturm 10**
**D-5068 Odenthal(DE)**
Erfinder: **Zeiler, Hans-Joachim, Dr.**
**Elsbeekerstrasse 46**
**D-5620 Velbert 15(DE)**
Erfinder: **Metzger, Karl Georg, Dr.**
**Pahlkestrasse 75**
**D-5600 Wuppertal 1(DE)**

(54) **Cyclopropylamine.**

㊼ Die Erfindung betrifft Cyclopropylamine, Zwischenprodukte zu deren Herstellung, Verfahren zu deren Herstellung und deren Verwendung zur Herstellung von 1-Cyclopropyl-chinoloncarbonsäuren.

EP 0 300 155 A2

## Cyclopropylamine

Die Erfindung betrifft Cyclopropylamine, Zwischenprodukte zu deren Herstellung, Verfahren zu deren Herstellung und deren Verwendung zur Herstellung von 1-Cyclopropyl-chinoloncarbonsäuren.

Die erfindungsgemäßen Verbindungen eignen sich zur Herstellung von neuen 7-Amino-1-(subst. cyclopropyl)-1,4-dihydro-4-oxo-3-chinolincarbonsäuren der Formel (I)

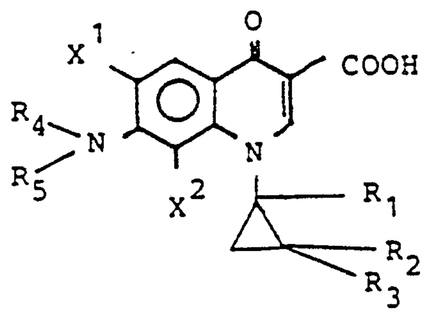

(I)

in welcher

$X^1$ und $X^2$ gleich oder verschieden sein können und für Wasserstoff, Halogen, insbesondere Chlor und Fluor stehen,

$R_1$, $R_2$ und $R_3$ für Wasserstoff, Methyl, Chlor und Fluor stehen, wobei niemals alle Reste $R_1$-$R_3$ gleich sind und

$R^4$ und $R^5$ gemeinsam mit dem Stickstoffatom, an das sie gebunden sind, einen 5- oder 6-gliedrigen heterocyclischen Ring bilden, der als Ringglied zusätzlich die Atome oder Gruppen -O-, -S-, -SO-, $-SO_2-$, $>N-R^6$ oder $-CO-\overset{|}{N}-R^6$ enthalten kann und der gegebenenfalls an den Kohlenstoffatomen ein- bis dreifach durch $C_1$-$C_4$-Alkyl, gegebenenfalls durch Chlor, Fluor, Brom, Methyl, Phenyl, Hydroxy, Methoxy, Benzyloxy, Nitro oder Piperidino ein- bis dreifach substituiertes Phenyl oder Cyclohexyl, 2-Thienyl, Hydroxy, Alkoxy mit 1 bis 3 Kohlenstoffatomen, Amino, Methylamino oder Ethylamino substituiert sein kann, wobei

$R^6$ für Wasserstoff, eine verzweigte oder unverzweigte Alkyl-, Alkenyl- oder Alkinylgruppe mit 1 bis 6 Kohlenstoffatomen, die gegebenenfalls durch eine oder zwei Hydroxy-, Alkoxy-, Alkylamino- oder Dialkylaminogruppen mit 1 bis 3 Kohlenstoffatomen für einen Alkylrest, die Cyangruppe, die Alkoxycarbonylgruppe mit 1 bis 4 Kohlenstoffatomen im Alkoholteil substituiert sein kann, eine gegebenenfalls im Phenylrest substituierte Phenylalkylgruppe mit bis zu 4 Kohlenstoffatomen im aliphatischen Teil, einen gegebenenfalls durch Hydroxy, Methoxy, Chlor oder Fluor ein- oder zweifach substituierten Phenacylrest, einen Oxoalkylrest mit bis zu 6 Kohlenstoffatomen steht, ferner einen Rest $COR^7$, CN oder $SO_2R^8$ bedeutet, wobei

$R^7$ Wasserstoff, gegebenenfalls durch 1 oder 2 Substituenten aus der Reihe Amino, Alkoxycarbonyl mit 1 bis 3 Kohlenstoffatomen im Alkylteil, Carboxy oder Alkoxy mit 1 bis 3 Kohlenstoffatomen oder Halogen wie Chlor, Brom, Fluor substituiertes geradkettiges oder verzweigtes Alkyl mit 1 bis 4 Kohlenstoffatomen, Alkoxy mit 1 bis 4 Kohlenstoffatomen, Amino, Alkylamino oder Dialkylamino mit 1 bis 5 Kohlenstoffatomen im Alkylteil und

$R^8$ geradkettiges oder verzweigtes Alkyl mit 1 bis 3 Kohlenstoffatomen darstellt, und deren pharmazeutisch verwendbare Hydrate, Säureadditionssalze, Alkali-, Erdalkali- und Guanidiniumsalze, die eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I) in denen

$X^1$ und $X^2$ gleich oder verschieden sein können und für Wasserstoff, Chlor und Fluor stehen und

$R_1$, $R_2$ und $R_3$ für Wasserstoff, Methyl, Chlor und Fluor stehen, wobei niemals alle Reste $R_1$ - $R_3$ gleich sind und

$R^4$ und $R^5$ gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, $-SO_2-$, $N-R^6$ oder $-CO-\overset{|}{N}-R^6$ enthalten kann und der gegebenenfalls an den Kohlenstoffatomen ein- bis zweifach durch $C_1$-$C_3$-Alkyl, Cyclohexyl, gegebenenfalls durch Chlor, Fluor, Brom, Methyl, Phenyl, Hydroxy, Methoxy, Benzyloxy, Nitro oder Piperidino ein-oder zweifach substituiertes Phenyl, 2-Thienyl, Hydroxy, Amino

oder Methylamino substituiert sein kann, wobei
$R^6$ für Wasserstoff, eine verzweigte oder unverzweigte Alkyl-, Alkenyl- oder Alkinylgruppe mit 1 bis 4 Kohlenstoffatomen, die gegebenenfalls durch eine oder zwei Hydroxygruppen substituiert sein kann, einen Phenacylrest, einen Oxoalkylrest mit bis zu 5 Kohlenstoffatomen sowie für einen Rest $COR^7$, steht, wobei
$R^7$ Wasserstoff, geradkettiges oder verzweigtes Alkyl mit 1 bis 3 Kohlenstoffatomen, Alkoxy mit 1 bis 3 Kohlenstoffatomen, Amino, Alkylamino oder Dialkylamino mit 1 bis 3 Kohlenstoffatomen im Alkylteil bedeutet.

Besonders bevorzugt sind diejenigen Verbindungen der Formel (I), in denen
$X^1$ und $X^2$ gleich oder verschieden sein können und für Wasserstoff, Chlor und Fluor stehen, und
$R_1$, $R_2$ und $R_3$ für Wasserstoff, Methyl, Chlor und Fluor stehen, wobei niemals alle Reste $R_1$-$R_3$ gleich sind und
$R^4$ und $R^5$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppen N-$R^6$ oder $-CO-\overset{|}{N}-R^6$ enthalten kann und der gegebenenfalls an den Kohlenstoffatomen ein- bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei
$R^6$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxoalkylrest mit bis zu 4 Kohlenstoffatomen sowie einen Rest $COR^7$ steht, wobei
$R^7$ Wasserstoff oder Alkyl mit ein oder zwei Kohlenstoffatomen bedeutet.

Weiterhin wurde gefunden, daß man die Verbindungen der Formel (I) erhält, wenn man die 1-(subst.cyclopropyl)-7-halogen-1,4-dihydro-4-oxo-3-chinolincarbonsäuren der Formel (II),

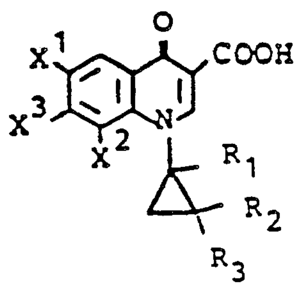

(II)

in welcher
$X^1$ und $X^2$ die oben angegebene Bedeutung haben, und
$X^3$ für Halogen, bevorzugt Chlor oder Fluor, steht, mit Aminen der Formel (III),

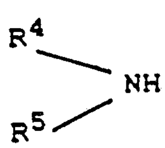

(III)

in welcher
$R^4$ und $R^5$ die oben angegebene Bedeutung haben,
gegebenenfalls in Gegenwart von Säurebindern umsetzt (Methode A).

Erfindungsgemäße Verbindungen der Formel (I) können auch erhalten werden, indem man eine 7-(1-Piperazinyl)-3-chinoloncarbonsäure der Formel (IV),

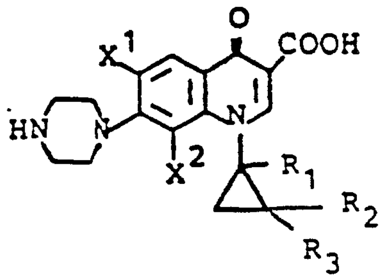

(IV)

in welcher
$X^1$ und $X^2$ die oben angegebene Bedeutung haben und
der Piperazinylrest an den Kohlenstoffatomen 1 - 3fach durch $C_1$-$C_4$-Alkyl, 2-Thienyl oder gegebenenfalls substituiertes Cyclohexyl oder Phenyl substituiert sein kann, mit Verbindungen der Formel (V)

$R^6X$ (V)

in welcher
$R^6$ die oben angegebene Bedeutung hat, jedoch nicht Wasserstoff sein kann, und
X Fluor, Chlor, Brom, Iod, Hydroxy, Acyloxy, Ethoxy, Phenoxy, 4-Nitrophenoxy bedeutet,
gegebenenfalls in Gegenwart von Säurebindern umsetzt (Methode B).

Man erhält erfindungsgemäße Verbindungen der Formel (I) auch, wenn man eine 7-(1-Piperazinyl)-3-chinoloncarbonsäure der Formel (IV), in welcher der Piperazinylrest an den Kohlenstoffatomen 1 - 3fach durch $C_1$-$C_4$-Alkyl, 2-Thienyl oder gegebenenfalls substituiertes Cyclohexyl oder Phenyl substituiert sein kann, mit Michael-Acceptoren der Formel (VI),

$B\text{-}CH=CH_2$ (VI)

in der B für CN, CO-$R^9$ oder COOR $^{10}$ steht,
wobei $R^9$ für Methyl oder Ethyl und
$R^{10}$ für Methyl, Ethyl, n- oder i-Propyl steht,
umsetzt (Methode C).

Verwendet man bei der Umsetzung nach Methode A 2-Methylpiperazin und 7-Chlor-6-fluor-1,4-dihydro-1-(1-methylcyclopropyl)-4-oxo-3-chinolincarbonsäure als Ausgangsstoffe, so kann der Reaktionsablauf durch das folgende Formelschema wiedergegeben werden:

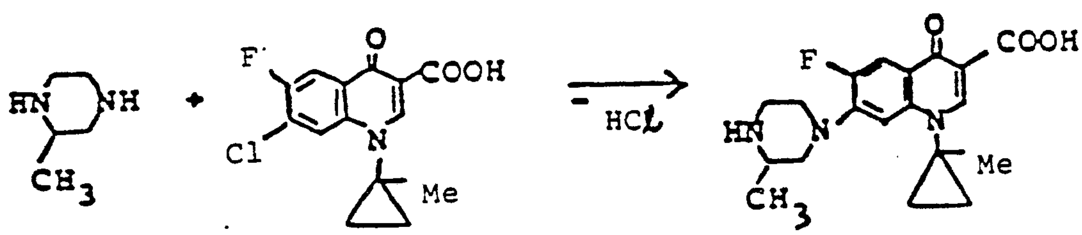


Verwendet man bei der Umsetzung nach Methode B Ethyliodid und 6-Fluor-1,4-dihydro-1-(1-methylcyclopropyl)-4-oxo-7-(1-piperazinyl)-3-chinolincarbonsäure als Ausgangsstoffe, so kann der Reaktionsablauf durch das folgende Formelschema wiedergegeben werden:

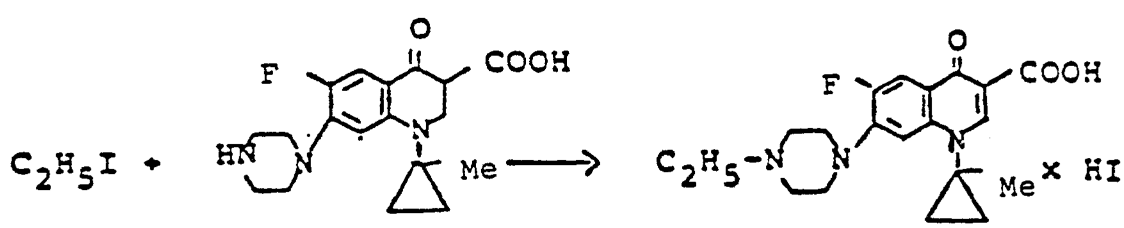

Verwendet man beispielsweise bei der Umsetzung von (IV) mit (V) nach Methode B 6-Fluor-1,4-dihydro-1-(1-methylcyclopropal)-7-(1-methylpiperazinyl)-4-oxo-3-chinolincarbonsäure und Ameisen-essigsäure-anhydrid als Ausgangsverbindungen, so kann der Reaktionsablauf durch das folgende Formelschema wiedergegeben werden:

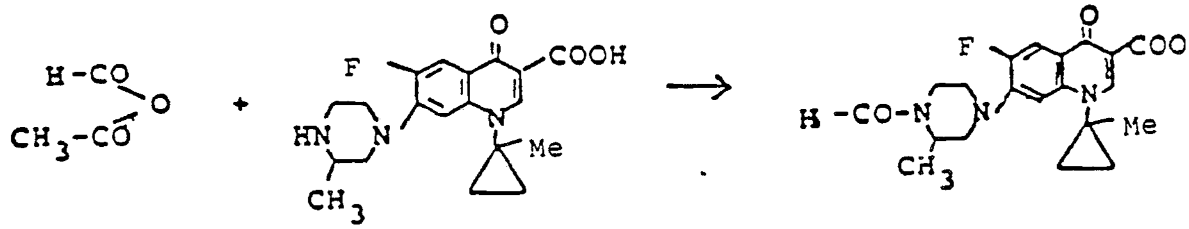

Verwendet man beispielsweise nach Methode C 6-Fluor-1.4-dihydro-1-(1-methylcyclopropyl)-4-oxo-7-(1-piperazinyl)-3-chinolincarbonsäure und Methylvinylketon als Ausgangsverbindungen, so kann der Reaktionsablauf durch das folgende Formelschema wiedergegeben werden:

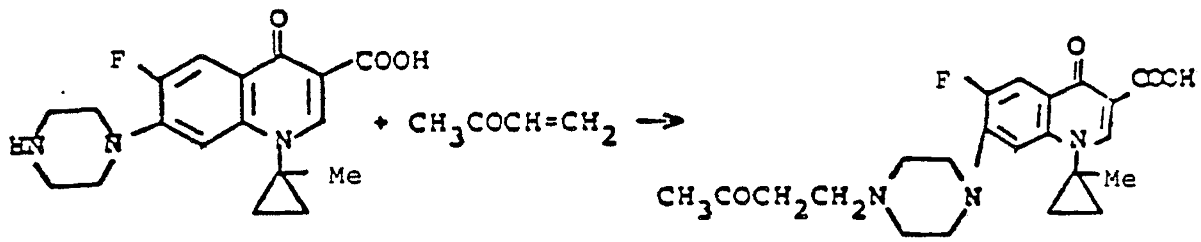

Die als Ausgangsstoffe nach Methode A verwendbaren 1-Cyclopropyl-6,7,8-trihalogen-1,4-dihydro-4-oxo-3-chinolincarbonsäuren der Formel (II) können gemäß folgendem Reaktionsschema hergestellt werden:

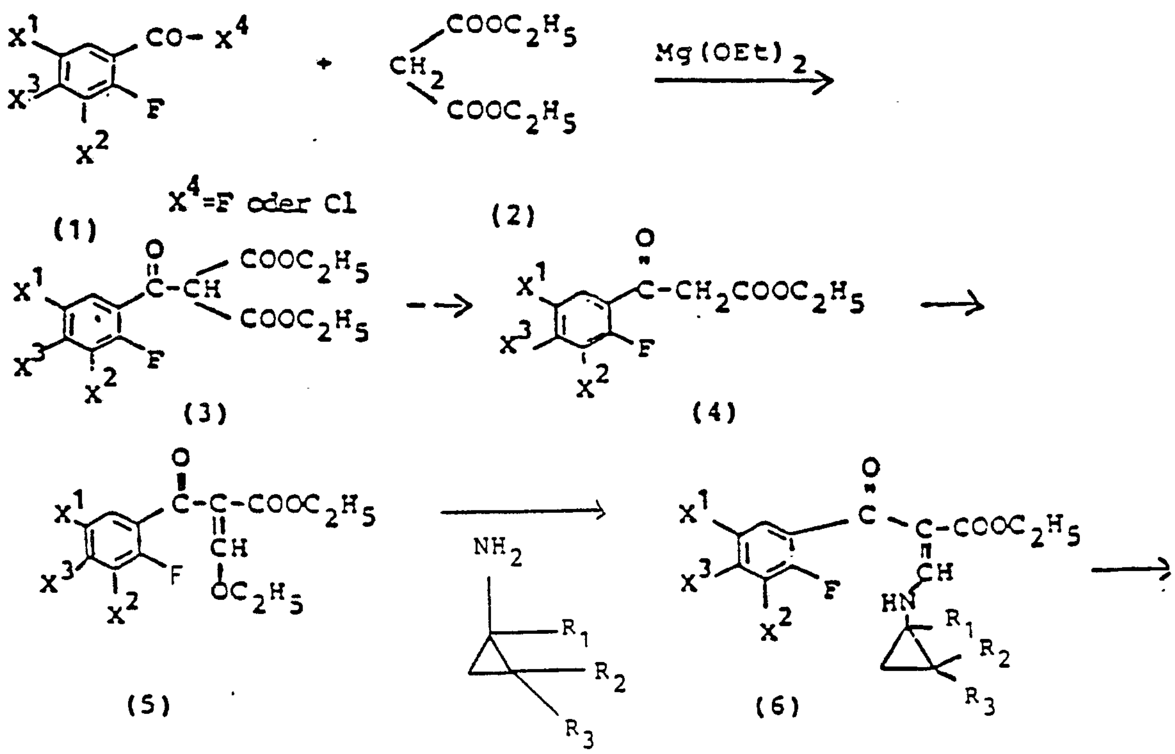


(1) (2) (3) (4) (5) (6)

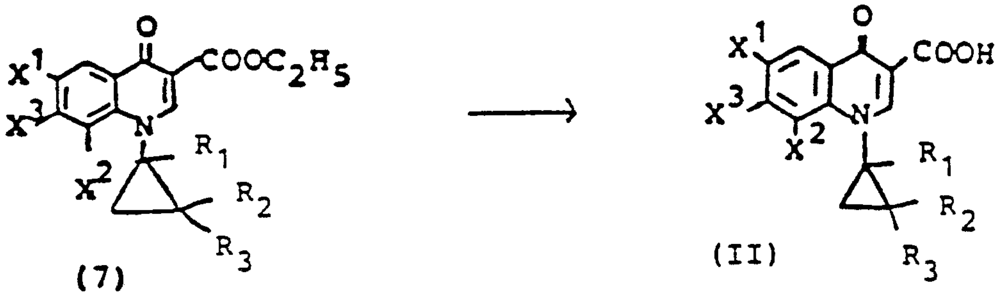

(7) (II)

Danach wird Malonsäurediethylester (2) in Gegenwart von Magnesiumethylat mit dem entsprechenden Benzoylfluorid bzw. -chlorid (1) zum Aroylmalonester (3) acyliert (Organicum, 3. Auflage 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit Orthoameisensäure-triethylester/Acetanhydrid in den 2-(2,3,4,5-Tetrahalogenbenzoyl)-3-ethoxy-acrylsäureethylester (5) übergeht. Die Umsetzung von (5) mit dem entsprechend substituierten Cyclopropylamin in einem Lösungsmittel, wie z. B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 °C, bevorzugt 80 bis 180 °C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid, Natrium- oder Kalium-carbonat und besonders bevorzugt Kalium-oder Natrium-fluorid in Betracht. Es kann vorteilhaft sein, einen Überschuß von 10 Mol-% an Base einzusetzen.

Die als Ausgangsstoffe für diesen Syntheseweg benötigten Benzoylhalogenide werden wie folgt hergestellt:

3,5-Dichlor-2,4-difluor-benzoylfluorid (Siedepunkt 97 °/20 mbar; $n_D^{20}$ = 1,5148) und 5-Chlor-2,3,4-trifluorbenzoylfluorid (Siedepunkt 66-70 °/20 mbar $n_D^{20}$ = 1,4764) erhält man nebeneinander beim Erhitzen von Tetrachlorbenzoylchlorid mit Kaliumfluorid im Sulfolan auf erhöhte Temperaturen:

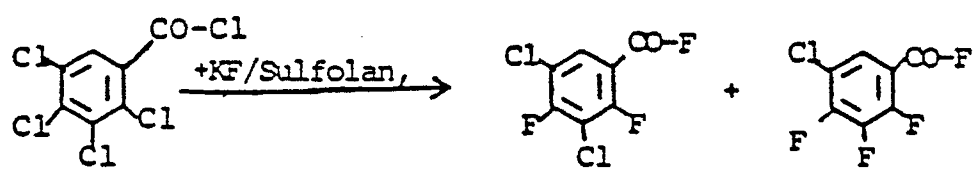


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94 °/18 mbar; $n_D^{20}$ = 1,5164) umgesetzt wird:

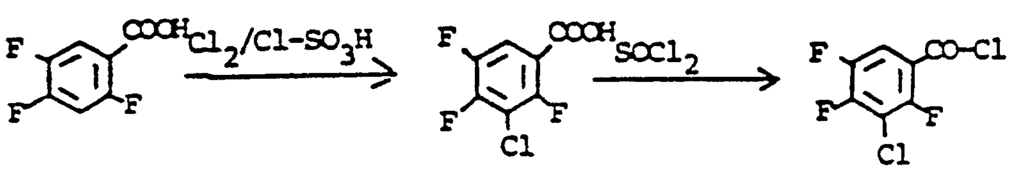


Die im letzten Schritt erfolgende Esterhydrolyse von (7) unter basischen oder sauren Bedingungen führt zu den 1-(subst. cyclopropyl)-1,4-dihydro-4-oxo-3-chinolincarbonsäuren.

Die als Ansgangsstoffe benötigten Cyclopropylamine sind teilweise bekannt.

Das eingesetzte 1-Amino-2,2-difluorcyclopropan wurde wie folgt dargestellt:

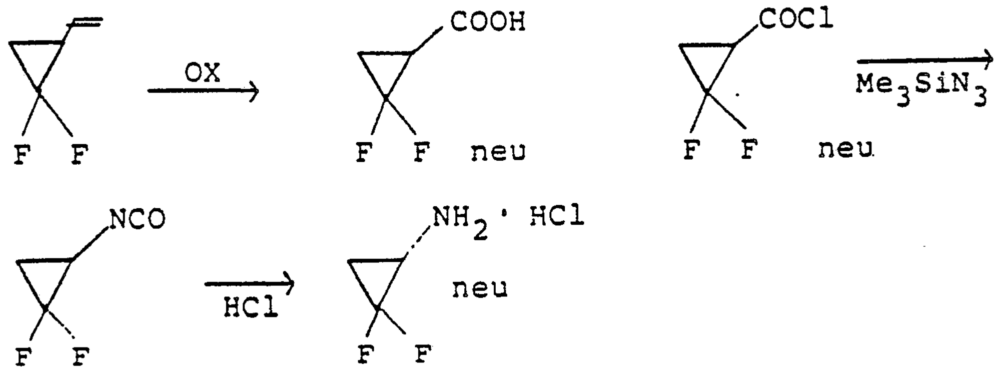


a) 2,2-Difluorcyclopropancarbonsäure

20,8 g 2,2-Difluorvinylcyclopropan werden in 220 ml Wasser vorgelegt. Zu der gut gerührten Emulsion werden portionsweise 96 g fein gepulvertes $KMnO_4$ so zugegeben, daß die Temperatur 35 °C nicht überschreitet. Nach einstündigem Rühren wird das $MnO_2$ abfiltriert. Die Mutterlauge versetzt man mit $NaHSO_3$ bis Entfärbung eingetreten ist. Danach wird mit verdünnter $H_2SO_4$ angesäuert. Man extrahiert mit $Et_2O$, trennt die organische Phase ab, trocknet und engt ein.

Ausbeute: 18 g

Schmlezpunkt: 60 - 61 °C.

b) 2,2-Difluorcyclopropancarbonsäurechlorid

18 g Difluorcyclopropancarbonsäure und 32,7 ml $SOCl_2$ werden 2 Stunden gekocht. Danach wird das überschüssige Thionylchlorid abdestilliert. Der Rückstand wird fraktioniert.

Ausbeute: 10 g

Siedepunkt: 110 - 112 °C.

c) 2,2-Difluorcyclopropylisocyanat

10 g des Säurechlorids werden zu einer Lösung von 19,5 g $Me_3SiN_3$ in 42 ml Toluol getropft. Man erhitzt auf 60-80 °C bis kein Gas mehr entsteht.

Für die Umsetzung zum Aminhydrochlorid wird die Lösung des Isocyanats in Toluol weiterverarbeitet.

Das Isocyanat kann also auch durch Destilliation rein gewonnen werden.

Siedepunkt: 64-65 °C

IR 2280 $cm^{-1}$ (NCO).

d) Zu der Lösung des Isocyanats in Toluol aus c) werden bei Raumtemperatur 60 ml konz. HCl gegeben. Die beiden Phasen werden geschüttelt, bis kein Gas mehr entsteht. Man trennt die wäßrige Phase ab und engt sie im Vakuum ein.

Ausbeute: 5,1 g 2,2-Difluorcyclopropylaminhydrochlorid

Schmelzpunkt: 125 °C.

Nach den gleichen Verfahren wird aus 2,2-Dichlorcyclopropancarbonsäure 1-Amino-2,2-dichlorcyclopropanhydrochlorid gewonnen.

2,2-Dichlorcyclopropylaminhydrochlorid

a) 2,2-Dichlorcyclopropancarbonsäurechlorid

70 g 2,2-Dichlorcyclopropancarbonsäure und 45,6 ml Thienylchlorid werden gekocht, bis die Gasentwicklung beendet ist. Danach wird überschüssiges Thionylchlorid abgezogen und der Rückstand destilliert.

Ausbeute: 77 g
Siedepunkt: 58-59 °C (12 mbar).

b) 2,2-Dichlorcyclopropylisocyanat

In 250 ml Tetrachlorkohlenstoff werden 60,3 g Trimethylsilylazid und 77 g Dichlorcyclopropancarbonsäurechlorid bis zur Beendigung der Gasentwicklung gekocht. Danach wird das Lösungsmittel im Vakuum entfernt. Der Rückstand wird destilliert.

Ausbeute: 44,3 g
Siedepunkt: 51-53 °C (10 mbar)
IR: 2230 $cm^{-1}$ (NCO).

c) 2,2-Dichlorcyclopropylaminhydrochlorid

22 g Isocyanat aus b) werden in 150 ml konz. HCl getropft. Anschließend wird eine Stunde auf 60 °C erwärmt. Danach dampft man im Vakuum bis zur Trockne ein.

Es werden 25 g der Titelverbindung erhalten.

Schmelzpunkt: 158 °C.

Die als Ausgangsstoffe verwendeten Amine (III) sind bekannt oder können nach literaturbekannten Verfahren erhalten werden [US 4 166 180, J. Med. Chem. 26, 1116 (1983)]. Durch katalytische Hydrierung werden aus den 2-Aryl-piperazinen die entsprechenden 2-Cyclohexyl-piperazine erhalten; z. B.: 2-Cyclohexyl-piperazin (wachsartig, Schmelzpunkt 71-73 °C). Als Beispiele seien genannt:
Morpholin, Piperidin, Thiomorpholin, Pyrrolidin, Piperazin, N-Methylpiperazin, N-Ethylpiperazin, N-(2-Hydroxyethyl)-piperazin, N-Formylpiperazin, 2-Methylpiperazin, 1,2-Dimethylpiperazin, cis- und trans-2,5-Dimethylpiperazin, cis- und trans-2,6-Dimethylpiperazin, 2-Ethylpiperazin, 2-Propylpiperazin, 2-Isopropylpiperazin, 2-Isobutylpiperazin, 2-Piperazinon, 1-Methyl-2-piperazinon, 1-Ethyl-2-piperazinon, 2-Cyclohexylpiperazin, 2-Phenylpiperazin, 2-(4-Chlorphenyl)-piperazin, 2-(4-Fluorphenyl)-piperazin, 2-(4-Bromphenyl)-piperazin, 2-(4-Methylphenyl)-piperazin, 2-(4-Biphenyl)-piperazin, 2-(4-Methoxyphenyl)-piperazin, 2-(4-Benzyloxyphenyl)-piperazin, 2-(4-Hydroxyphenyl)-piperazin, 2-(4-Nitrophenyl)-piperazin, 2-(3-Nitrophenyl)-piperazin, 2-(4-Piperidinophenyl)-piperazin, 2-(3,4-Dimethoxyphenyl)-piperazin, 2-(3,4,5-Trimethoxyphenyl)-piperazin, 2-(3,4-dimethoxy-6-methyl)-piperazin, 2-(2-Thienyl)-piperazin, 3-Aminopyrrolidin.

Die als Ausgangsstoffe verwendeten Verbindungen der Formel (V) sind bekannt. Als Beispiele seien genannt:
Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butyl chlorid, n-Pentylchlorid, 3-Methylbutylchlorid, n-Hexylbromid, Ameisensäureessiganhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, N-(tert.-Butoxycarbonyl)-glycin-4-nitrophenylester, N-(tert.-Butoxycarbonyl)-L-alanin-4-nitro-phenylester, N-(tert.-Butoxycarbonyl)-L-leucin-4-nitrophenylester, N-(tert.-Butoxycarbonyl)-L-valin-4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethylcarbamidsäurechlorid, Methansulfonsäurechlorid, Ethansulfonsäurechlorid, Propan-1-sulfonsäurechlorid, Ameisensäure.

Die erfindungsgemäß verwendbaren Verbindungen der Formel (VII) sind bekannt. Als Beispiele seien genannt:
Acrylnitril, Methylvinylketon, Acrylsäuremethylester, Acrylsäureethylester.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können als üblichen anorganischen und organischen Säurebindungsmittel verwendet werden. Hierzu gehören vorzugsweise die Alkalihydroxide, Alkalicarbonate, organischen Amine und Amidine. Als besonders geeignet seien im einzelnen genannt: Triethylamin, 1,4-Diaza-bicyclo[2,2,2]-octan (DABCO), 1,8-Diaza-bicyclo[5,4,0]-undec-7-en(DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]octan (DABCO) oder 1,8-Diazabicyclo-[5,4,0] undec-7-en (DBU).

Die Reaktionstemperaturen können in einem größeren Bereich variiert werden. Im allgemeinen arbeitet man zwischen etwa 20 und etwa 180 °C, vorzugsweise zwischen 40 und 110 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol, der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20 °C und etwa 150 °C, vorzugsweise zwischen 50 °C und 100 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der Verbindung (IV) 1 bis 5 Mol, vorzugsweise 1 bis 2 Mol, der Verbindung (VI) ein.

Außer den in den Beispielen aufgeführten Verbindungen seien als neue Wirkstoffe im einzelnen genannt:

8-Chlor-6-fluor-1,4-dihydro-1-(1-methylcyclopropyl)-4-oxo-7-(1-piperazinyl)-3-chinolincarbonsäure
6-Chlor-8-fluor-1,4-dihydro-1-(1-methylcyclopropyl)-4-oxo-7-(1-piperazinyl)-3-chinolincarbonsäure
6-Chlor-8-fluor-1-(2,2-difluorcyclopropyl)-1,4-dihydro-4-oxo-7-(1-piperazinyl)-3-chinolincarbonsäure
6,8-Difluor-1-(2-fluorcyclopropyl)-1,4-dihydro-4-oxo-7-(1-piperazinyl)-3-chinolincarbonsäure
1-(2,2-Dichlorcyclopropyl)-6,8-difluor-1,4-dihydro-4-oxo-7-(1-piperazinyl)-3-chinolincarbonsäure
8-Chlor-6-fluor-1,4-dihydro-1-(2-methylcyclopropyl)-7-(3-methyl-1-piperazinyl)-4-oxo-3-chinolincarbonsäure
8-Chlor-6-fluor-1,4-dihydro-1-(1-methylcyclopropyl)-7-(3-methyl-1-piperazinyl)-4-oxo-3-chinolincarbonsäure
6-Chlor-8-fluor-1-(2-fluorcyclopropyl)-1,4-dihydro-4-oxo-7-(1-piperazinyl)-3-chinolincarbonsäure
1-(2-Chlorcyclopropyl)-6,8-difluor-1,4-dihydro-4-oxo-7-(1-piperazinyl)-3-chinolincarbonsäure
6,8-Difluor-1,4-dihydro-1-(1-methylcyclopropyl)-4-oxo-7-(3-phenyl-1-piperazinyl)-3-chinolincarbonsäure
8-Chlor-6-fluor-1,4-dihydro-1-(1-methylcyclopropyl)-4-oxo-7-(3-phenyl-1-piperazinyl)-3-chinolincarbonsäure
6,8-Difluor-1-(2,2-difluorcyclopropyl)-1,4-dihdyro-4-oxo-7-(3-phenyl-1-piperazinyl)-3-chinolincarbonsäure
8-Chlor-6-fluor-1-(2,2-difluorcyclopropyl)-1,4-dihydro-7-(4-methyl-1-piperazinyl)-4-oxo-3-chinolincarbonsäure
6-Chlor-8-fluor-1-(2,2-difluorcyclopropyl)-1,4-dihydro-7-(4-methyl-1-piperazinyl)-4-oxo-3-chinolincarbonsäure
7-(4-Acetyl-1-piperazinyl)-6,8-difluor-1,4-dihydro-1-(1-methylcyclopropyl)-4-oxo-3-chinolincarbonsäure
6,8-Difluor-1,4-dihydro-4-oxo-7-[4-(3-oxo-butyl)-1-piperazinyl]-3-chinolincarbonsäure
1-(2,2-Dichlorcyclopropyl)-6-fluor-1,4-dihydro-4-oxo-7-(1-piperazinyl)-3-chinolincarbonsäure

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z. B. Penicilline, Cephalosporine, Aminoglykoside, Sulfonamide, Tetracycline.

Diese wertvollen Eigenschaften ermöglichen ihre Verwendung als chemotherapeutische Wirkstoffe in

der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-positive und gram-negative Bakterien sowie bakterienähnliche Mikroorganismen bekämpft und die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden:
Gram-positive Kokken, z. B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrheae) sowie gram-negative Stäbchen wie Enterobakteriaceen, z. B. Escherichia coli, Hämophilus influenzae, Citrobacter (Citrob. freundii, Citrob. diverms), Salmonella, Shigella; ferner Klebsiellen (Klebsiella pneumoniae, Klebs. oxytoca), Enterobacter (Ent. aerogenes, Ent. agglomerans), Hafnia, Serratia (Serr. mascescens), Proteus (Pr. nurabilis, Pr. rettgeri, Pr. vulgaris) Providencia, Yersinia sowie die Gattung Acinetobacter. Darüberhinaus umfaßt das antibakterielle Spektrum die Gattung Pseudomonas (Ps. aeruginosa, Ps. maltophilia) sowie strikt anaerobe Bakterien wie z. B. Bacteroides fragilis, Vertreter der Gattung Peptococcus, Peptostreptococcus sowie die Gattung Clostridium; ferner Mykoplasmen (M. pneumoniae, M.hominis, M.urealyticum) sowie Mykobakterien, z. B. Mycobacterium tuberculosis.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:
Otitis; Pharyngitis; Pneumonie; Peritonitis; Pyelonephritis; Cystitis; Endocarditis; Osteomyelitis; Bronchitis; Arthritis; lokale Infektionen; septische Erkrankungen.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegt, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Paraffin und (f) Resorptionsbeschleuniger, z.B. quartäre Ammoniumverbindungen, (g) Netzmittel, z. B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykol DAB | 2,0 mg |
| Titan-(IV)-oxid | 2,0 mg |
| | 10,0 mg |

Der oder die Wirkstoffe können gegebenenfalls mit einem oder mehreren der oben aufgeführten Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z.B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokistalline Cellulose, Aluminiummetahydroxid, Bentonit, Agar-Agar und Tragant oder Gemische dieser Stoffe enhalten.

Die genannten Formulierungsformen können auch Färbemittel, Konservierungsstoffe sowie geruchs-

und geschmacksverbesserte Zusätze, z. B. Pfefferminzöl und Eukalyptusöl und Süßmittel, z. B. Saccharin, enthalten.

Die therapeutisch wirksamen Vervindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können 10kal, oral, parenteral, interperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführten Wirkstoffmengen überschritten werden müssen. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle 1 sind die MHK-Werte angegeben.

MHK mcg/ml

| Stamm | Beisp.Nr. 1 | 3 | Norfloxacin |
|---|---|---|---|
| E.coli Neumann | 0.06 | 0.06 | 0.06 |
| E.coli T 7 | ≤0.015 | 0.03 | 0.06 |
| E.coli A 261 | ≤0.015 | 0.03 | 0.03 |
| Klebsiella 8085 | 0.03 | 0.06 | 0.125 |

Agardilutionstest (Isosensitest-Medium);
Denley Multipointinoculator

Die folgenden Beispiele erläutern die Erfindung:

Beispiel A

7-Chlor-6-fluor-1,4-dihydro-1-(1-methylcyclopropyl)-4-oxo-3-chinolincarbonsäure

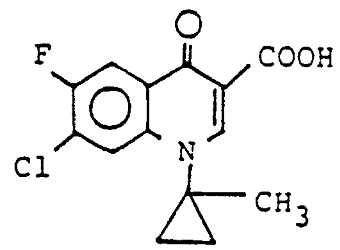

25,2 g 2-(2,4-Dichlor-5-fluorbenzoyl)-3-ethoxy-acrylsäureethylester werden in 30 ml EtOH vorgelegt. Unter Kühlung werden 5,7 g 1-Amino-1-methylcyclopropan in 30 ml EtOH zugetropft. Man rührt noch eine Stunde bei Raumtemperatur und gibt dann 70 ml Eiswasser zu. Der Niederschlag wird abgesaugt, mit wässrigem Ethanol gewaschen und getrocknet.
Ausbeute: 25,4 g 2-(2,4-Dichlor-5-fluorbenzoyl)-3-(1-methyl-cyclopropylamino)-acrylsäureethylester vom Schmelzpunkt 81-82° C.

25 g dieser Verbindung werden mit 10 g $K_2CO_3$ in 100 ml DMF zwei Stunden auf 140° C erhitzt. Dann gießt man heiß auf Eis, isoliert den Niederschlag und wäscht mit Wasser.
Ausbeute: 21,1 g 7-Chlor-6-fluor-1,4-dihydro-1-(1-methylcyclopropyl)-4-oxo-3-chinolincarbonsäureethylester. Fp 218-19° C.

21,1 dieser Verbindung werden in einem Gemisch aus 75 ml Essigsäure, 57 ml Wasser und 7 ml Schwefelsäure 1,5 Stunden auf 150° C erhitzt. Danach tropft man unter Eiskühlung 120 ml Wasser zu, saugt den Niederschlag ab und wäscht mit Wasser.
Ausbeute: 18,2 g 7-Chlor-6-fluor-1,4-dihydro-1-(1-methylcyclopropyl)-4-oxo-3-chinolincarbonsäure vom Schmelzpunkt 301-3° C.

Beispiel B

7-Chlor-6-fluor-1,4-dihydro-1-(2-methylcyclopropyl)-4-oxo-3-chinolincarbonsäure

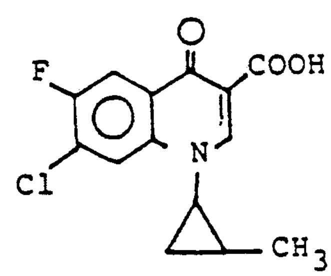

Man setzt 2-(2,4-Dichlor-5-fluorbenzoyl)-3-ethoxyacrylsäureethylester mit 1-Amino-2-methylcyclopropan analog Beispiel A um.

Es werden folgende Stufen erhalten:
2-(2,4-Dichlor-5-fluorbenzoyl)-3-(2-methylcyclopropyl)-aminoacrylsäureethylester (Ausbeute 91 %; Schmelzpunkt 80-85° C)
7-Chlor-6-fluor-1,4-dihydro-1-(2-methylcyclopropyl)-4-oxo-3-chinolincarbonsäureethylester (Ausbeute 84 %; Schmelzpunkt 185-8° C)
7-Chlor-6-fluor-1,4-dihydro-1-(2-methylcyclopropyl)-4-oxo-3-chinolincarbonsäure (Ausbeute 79 %; Schmelzpunkt 218-20° C)

Beispiel C

6,7,8-Trifluor-1-(2,2-difluorcyclopropyl)-1,4-dihydro-4-oxo-3-chinolincarbonsäure.

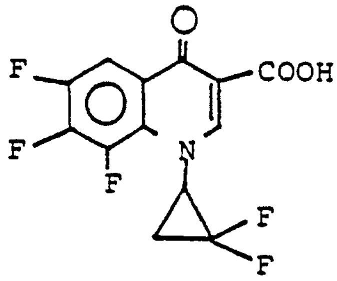

6 g 3-Ethoxy-2-(2,3,4,5-tetrafluorbenzoyl)-acrylsäureethylester (bekannt) in 30 ml $CH_2Cl_2$ und 2,4 g 1-Amino-2,2-difluorcyclopropan hydrochlorid in 12 ml Wasser werden zusammen bei Eiskühlung vorgelegt. Unter intensivem Rühren wird eine Lösung von 1,6 g $NaHCO_3$ in 20 ml Wasser zugetropft. Man rührt 2 Stunden nach, trennt die Phasen, wäscht die org. Phase mit Wasser, trocknet und engt ein. Es bleiben 6,2 g roher 2-(2,3,4,5-Tetrafluorbenzoyl)-3-(2,2-difluorcyclopropylamino)-acrylsäureethylester zurück.
Ausbeute: 92 %

3,7 g dieser Verbindung werden mit 0,7 g NaF in 22 ml DMF 2 Stunden gekocht. Danach gießt man auf Eis und isoliert das ausgefallene Produkt.
Ausbeute: 3,0 g (86 %)
6,7,8-Trifluor-1-(2,2-difluorcyclopropyl)-1,4-dihydro-4-oxo-3-chinolincarbonsäureethylester.
Schmelzpunkt: 118-20 °C

4,5 g dieser Verbindung werden in einem Gemisch aus 14 ml AcOH, 11 ml Wasser und 1,3 ml Schwefelsäure 2 Stunden auf 150 ° (Bad) erhitzt. Nach Abkühlen gießt man auf Eis und isoliert den Niederschlag.
Ausbeute: 3,0 g 6,7,8-Trifluor-1-(2,2-difluorcyclopropyl)-1,4-dihydro-4-oxo-3-chinolincarbonsäure (72 %)
Schmelzpunkt: 238-40 ° (Z)

Beispiel 1

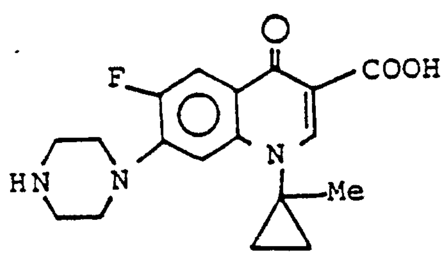

3 g Beispiel A und 2,6 g wasserfreies Piperazin werden in 75 ml Pyridin 8 Stunden gekocht. Danach wird das Gemisch im Vakuum eingeengt. Der Rückstand wird mit 45 ml Wasser verrührt und mit HCl auf pH 5 gestellt. Man saugt den Niederschlag ab, wäscht mit Wasser und trocknet bei 100 °C im Vakuum.
Ausbeute: 2,1 g 6-Fluor-1,4-dihdyro-1-(1-methylcyclopropyl)-4-oxo-7-(1-piperazinyl)-3-chinolincarbonsäure
Schmelzpunkt: 269-71 °C

Analog Beispiel 1 erhält man folgende in 7-Stellung substituierte 7-Fluor-1,4-dihydro-1-(1-methylcyclopropyl)-4-oxo-3-chinolincarbonsäuren:

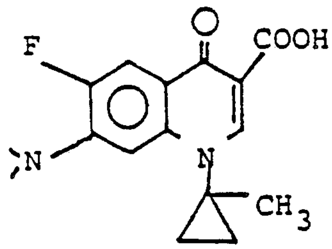

| Beispiel | 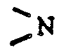 | Schmelzpunkt |
|---|---|---|
| 2 | 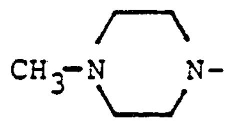 | 248-9°C |
| 3 | 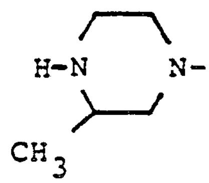 | 186-8°C |
| 4 | 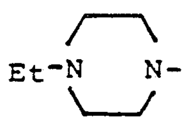 | 234-36°C |

| Beispiel | >N | Schmelzpunkt |
|---|---|---|
| 5 | 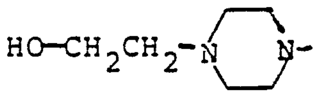 | 198-203°C |
| 6 | 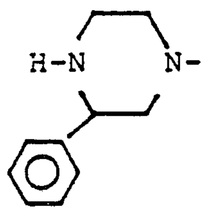 | 218-20°C |
| 7 | 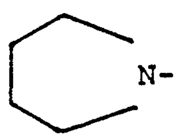 | 292-94°C |

<u>Beispiel 8</u>

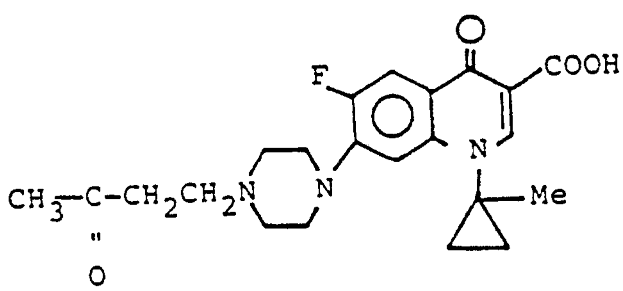

3,4 g des Produktes aus Beispiel 1 und 3,9 g Methylvinylketon werden in 25 ml Ethanol 6 Stunden gekocht. Es wird heiß filtriert und nach Abkühlen abgesaugt.
Ausbeute: 3,2 g 7-Fluor-1,4-dihydro-4-oxo-7-[4(3-oxo-butyl)-1-piperazinyl]-3-chinolincarbonsäure
Schmelzpunkt: 158-60 °C

Beispiel 9

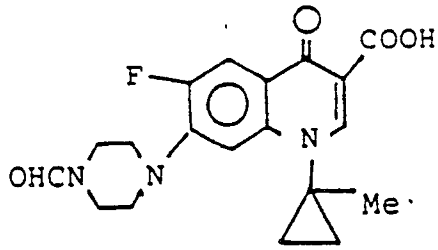

3,1 g des Produktes aus Beispiel 1 und 3 ml Ameisensäure werden in 30 ml DMF 8 Stunden gekocht. Nach Abkühlen wird der Niederschlag abgesaugt, mit Wasser und Methanol gewaschen und getrocknet.
Ausbeute: 2,0 g 6-Fluor-7-(4-formyl-1-piperazinyl)-1,4-dihydro-4-oxo-3-chinolincarbonsäure
Schmelzpunkt: 297-99° (Z)

Beispiel 10

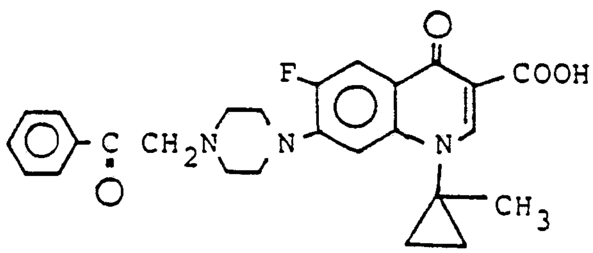

3,4 g des Produktes aus Beispiel 1 und 2,3 g ω-Chloracetophenon und 2,1 g Triethylamin werden in 50 ml Ethanol 9 Stunden gekocht. Man engt im Vakuum ein und verrührt mit Wasser. Der Niederschlag wird isoliert, getrocknet und in Toluol gelöst. Durch Zugabe von Leichtbenzin fällt das Produkt aus.
Ausbetue: 1 g 6-Fluor-1,4-dihydro-1-(1-methylcyclopropyl)-4-oxo-7-[4-(2-oxo-2-phenyl-ethyl)-1-piperazinyl]-3-chinolincarbonsäure
Schmelzpunkt: 215-18°

Beispiel 11

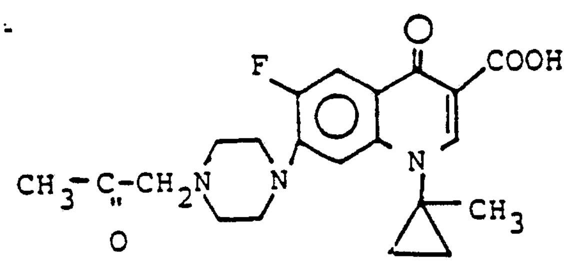

3,4 g des Produktes aus Beispiel 1, 3,9 g Chloraceton und 5,8 ml Triethylamin werden in 50 ml Ethynol 6 Stunden gekocht. Anschließend engt man ein, löst den Rückstand in MeOH und fällt mit Wasser aus.
Ausbeute: 2,0 g 7-Fluor-1,4-dihydro-1-(1-methyl-cyclopropl)-4-oxo-7-[4(2-oxo-propyl)-1-piperazinyl]-3-chinolincarbonsäure
Schmelzpunkt: 188-90° (Z)

Beispiel 12

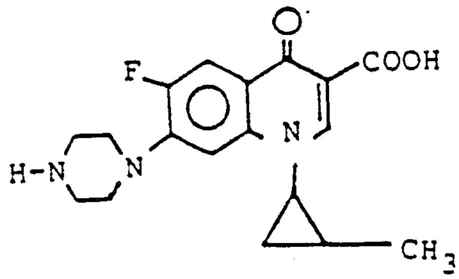

3 g Beispiel B und 2,6 g Piperazin werden in 75 ml Pyridin 8 Stunden gekocht. Man engt das Gemisch ein und verrührt den Rückstand mit 45 ml Wasser. Es wird mit HCl auf pH 5 gestellt. Der Niederschlag wird abgesaugt, mit Wasser gewaschen und getrocknet. Man erhält 1,8 g 6-Fluor-1,4-dihydro-1-(2-methylcyclopropyl)-4-oxo-7-(1-piperazinyl)-3-chinolincarbonsäure.
Schmelzpunkt: 110-20° C

Analog Beispiel 12 erhält man folgende in 7-Stellung substituierte 7-Fluor-1,4-dihydro-1-(2-methylcyclopropyl)-4-oxo-3-chinolincarbonsäuren:

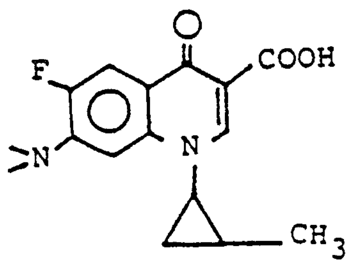

| Beispiel | 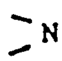 | Schmelzpunkt |
|---|---|---|
| 13 | 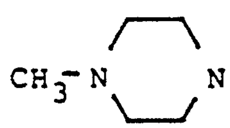 | 204-7° |
| 14 | 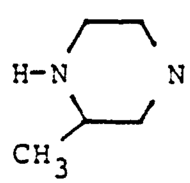 | 210-12° |
| 15 | 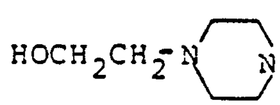 | 188-90° |

Beispiel 16

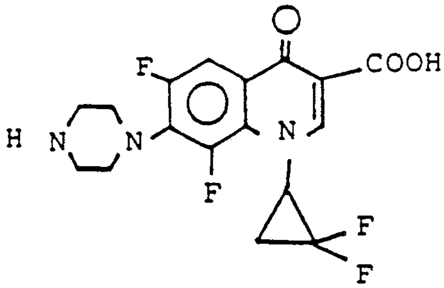

0,4 g Beispiel C und 0,54 g Piperazin werden in 2 ml DMSO 2 Stunden gekocht. Danach destilliert man das DMSO ab und verrührt den Rückstand mit Wasser. Der Niederschlag wird abgesaugt, gewaschen und getrocknet.

Ausbeute: 0,2 g 6,8-Difluor-1-(2,2-difluorcyclopropyl)-1,4-dihydro-4-oxo-7-(1-piperazinyl)-3-chinolincarbonsäure

Schmelzpunkt: 204-6 °C

Analog Beispiel 16 erhält man folgende in 7-Stellung substituierte 6,8-Difluor-1-(2,2-difluorcyclopropyl)-1,4-dihydro-4-oxo-3-chinolincarbonsäuren:

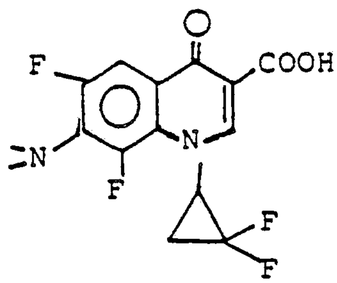

| Beispiel | >N | Schmelzpunkt |
|---|---|---|
| 17 | 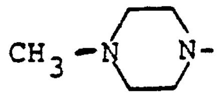 | 302-4°(Z) |
| 18 | 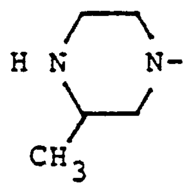 | 278-80°(Z) |
| 19 | 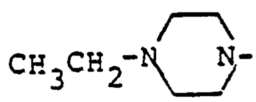 | 310°(Z) |

## Ansprüche

1. Verbindungen der Formel

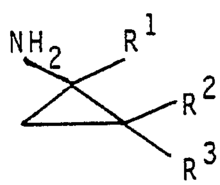

worin
$R^1$, $R^2$ und $R^3$ für Wasserstoff, Methyl, Chlor und Fluor stehen, wobei niemals alle Reste $R^1$ bis $R^3$ gleich sind.

2. 1-Amino-2,2-difluorcyclopropanhydrochlorid.
3. 1-Amino-2,2-dichlorcyclopropanhydrochlorid.
4. 2,2-Difluorcyclopropancarbonsäure.
5. 2,2-Difluorcyclopropancarbonsäurechlorid.
6. 2,2-Difluorcyclopropancarbonsäureazid.
7. 2,2-Difluorcyclopropanisocyanat.
8. 2,2-Dichlorcyclopropancarbonsäure.